# EUROPEAN PATENT SPECIFICATION

(11) **EP 0 568 350 B1**
(45) Date of publication and mention of the grant of the patent: **02.01.1997**
(21) Application number: 93303332.6
(22) Date of filing: 28.04.1993
(51) Int. Cl.: C09K 5/04, C10M 105/32

(54) **Liquid compositions containing blends of carboxylic esters**
Flüssige Zusammensetzungen enthaltend Mischungen von Karboxylsäureestern
Compositions liquides contenant des mélanges d'esters carboxyliques

(30) Priority: 28.04.1992 US 874894
(43) Date of publication of application: 03.11.1993
(73) Proprietor: The Lubrizol Corporation, Wickliffe, Ohio 44092 (US)
(72) Inventor: Davis, Kirk Emerson, Chester Township, Ohio 44026 (US)
(74) Representative: Mallalieu, Catherine Louise

(56) References cited:
- EP-A- 0 415 778
- EP-A- 0 435 253
- EP-A- 0 440 069

## Description

This invention relates to liquid compositions comprising at least one fluorine-containing hydrocarbon, and at least one lubricant. More particularly, the invention relates to liquid compositions useful as refrigeration liquids.

Chlorofluorocarbon, generally referred to in the industry as CFCs, use has been diminishing in recent years because of demands of environmentalists for the reduction if not a complete ban on the use of CFCs because of the detrimental effect of CFCs on the stratosphere's ozone layer. Examples of CFCs include CFC-13 which is chlorotrifluoromethane, CFC-12 which is dichlorodifluoromethane, and CFC-113 which is 1,2,2-trifluoro-1,1,2-trichloroethane.

The problem of finding a safe replacement for CFC refrigerants and foam-blowing agents has been difficult to solve. Several replacement candidates have been suggested as alternatives to the fully halogenated hydrocarbons, and these include halogenated hydrocarbons containing at least some hydrogen atoms such as HCFC-22 which is difluorochloromethane, HCFC-123 which is 1,1-dichloro-2,2,2-trifluoroethane, HFC-134a which is 1,1,1,2-tetrafluoroethane and HCFC-141b which is 1,1-dichloro-1-fluoroethane.

The ozone depletion potential of these proposed substitutes is significantly less than the ozone depletion potential of the previously used CFCs. The ozone depletion potential is a relative measure of the capability of the material to destroy the ozone layer in the atmosphere. HCFC-22 and HFC-134a generally are recommended as being candidates in refrigerant applications, and HFC-134a is particularly attractive because its ozone depletion potential has been reported as being zero.

In order for any of the replacement materials to be useful as refrigerants, the materials must be compatible with the lubricant utilized in the compressor. The presently used refrigerants such as CFC-12 are readily compatible with mineral lubricating oils which are utilized as the lubricant in air-conditioner compressors. The above-described refrigerant candidates, however, have different solubility characteristics than the refrigerants presently in use. For example, mineral lubricating oil is incompatible (i.e., insoluble) with HFC-134a. Such incompatibility results in unacceptable compressor life in compression-type refrigeration equipment including refrigerators and air-conditioners including auto, home, commercial, and industrial air-conditioners.

In order to perform as a satisfactory refrigeration liquid, the mixture of refrigerant and lubricant must be compatible and stable over a wide temperature range such as from about 0°C and above 80°C. For some uses, it is generally desirable for the lubricants to be soluble in the refrigerant at concentrations corresponding to the ratios customary in the environment of use, e.g. about 5 to 15%, over a temperature range from -30°, or preferably -40°C, or below, to 80°C or above. In addition to thermal stability, the refrigeration liquids must have acceptable viscosity characteristics which are retained even at high temperatures, and the refrigeration liquid should not have a detrimental effect on materials used as seals in the compressors.

Compositions comprising a tetrafluoroethane and polyoxyalkylene glycols are discussed in U.S. Patent 4,755,316. The compositions are useful in refrigeration systems. Refrigeration oils are described in U.S. Patents 4,248,726 and 4,267,064 which comprise mixtures of a polyglycol and 0.1 to 10% of glycidyl ether type epoxy compounds, or epoxidized fatty acid monoesters, and optionally, epoxidized vegetable oil. The lubricating oils are reported to be useful in refrigerators using a halogen-containing refrigerant such as Freons 11, 12, 13, 22, 113, 114, 500 and 502 (available from DuPont), and in particular with Freon 12 or 22.

U.S. Patent 4,431,557 describes fluid compositions comprised of a fluoro- and chloro-containing refrigerant, a hydrocarbon oil, and an alkylene oxide additive compound which improves the thermal resistance of the oil in the presence of the refrigerant. Examples of hydrocarbon oils include mineral oil, alkyl benzene oil, dibasic acid ester oil, polyglycols, etc. The composition may contain other additives including load-carrying additives such as phosphorus acid esters, phosphoric acid esters, etc. Examples of fluorocarbon refrigerants include R-11, R-12, R-113, R-114, R-500, etc.

U.S. Patent 4,428,854 describes absorption refrigerant compositions for use in refrigeration systems comprising 1,1,1,2-tetrafluoroethane and an organic solvent capable of dissolving the ethane. Among the solvents disclosed are organic amides, acetonitrile, N-methyl pyrroles, N-methyl pyrrolidine, N-methyl-2-pyrrolidone, nitromethane, various dioxane derivatives, glycol ethers, butyl formate, butyl acetate, diethyl oxalate, diethyl malonate, acetone, methyl ethyl ketone, other ketones and aldehydes, triethyl phosphoric triamide, triethylene phosphate, triethyl phosphate, etc.

Stabilized absorption compositions comprising (a) a halogenated hydrocarbon refrigerant, (b) a liquid absorbent of a polyethylene glycol methyl ether, and (c) at least one stabilizer are described in U.S. Patent 4,454,052. Examples of stabilizers include phosphate esters, epoxy compounds, and organotin compounds. The polyethylene glycol methyl ether-type compounds are of the general formula

CH₃-O-(CH₂H₄O)ₙR

wherein n is an integer of 1 to 6, and R is H, CH₃- or CH₃CO-. A variety of halogenated hydrocarbons are described including difluoromethane, 1,1,1,2-tetrafluoroethane, etc.

U.S. Patent 4,559,154 relates to absorption heat pumps utilizing as working fluid, a saturated fluorohydrocarbon or fluorohydrocarbon ether having from 3 to 5 carbon atoms. Solvents reported to be useful with such fluorohydrocarbons include ethers such as tetraglyme, amides which can be lactams such as the N-alkyl pyrrolidones, sulfonamides and ureas including cyclic ureas.

### Summary of the Invention

According to one aspect of the present invention there is provided a liquid composition comprising (A) at least one fluorine-containing hydrocarbon containing one to three carbon atoms; and a lubricant comprising (B) a blend of (1) at least one carboxylic ester of (I) a neo hydroxy compound selected from the group consisting of (i) a neo hydroxy compound with one to four hydroxyl groups, and (ii) a mixture of neo hydroxy compounds having an average of less than 3.5 hydroxyl groups per hydroxy compound, and (II) a monocarboxylic acylating agent selected from the group consisting of (i) a monocarboxylic acylating agent having four or five carbon atoms; and (ii) a combination of (a) a monocarboxylic acylating agent having four or five carbon atoms and (b) a monocarboxylic acylating agent having from 7 to 15 carbon atoms, provided that greater than 55% of the ester groups of (1) are derived from monocarboxylic acylating agent having four or five carbon atoms, and (2) at least one carboxylic ester of (III) a neo hydroxy compounds group consisting of (iii) a neo hydroxy compound having five or more four hydroxyl groups per hydroxy compound, and (iv) a mixture of neo hydroxy compounds having an average greater than 4.5 hydroxyl groups per hydroxy compound, and (IV) a combination of (a) a monocarboxylic acylating agent having four or five carbon atoms and (b) a monocarboxylic acylating agent having from 7 to 15 carbon atoms, provided that greater than 55% of the ester groups of (2) are derived from (IVb).

The liquid compositions may additionally contain (C) at least one additive selected from the group consisting of an alkyl phosphite, an alkyl phosphonic acid ester, a nitrogen-containing heterocycle, and a mixture thereof. Liquid compositions also are described wherein the fluorine-containing hydrocarbons also contain other halogens such as chlorine.

According to another aspect of the present invention there is provided a liquid composition comprising:
(A) at least one fluorine-containing hydrocarbon containing one to three carbon atoms and wherein fluorine is the only halogen present; and a lubricant comprising (B) an ester blend of
(1) at least one carboxylic ester of (I) a trimethylolalkane, and (II) a combination of (a) a monocarboxylic acylating agent having four or five carbon atoms and (b) a branched aliphatic monocarboxylic acylating agent having from 7 to 15 carbon atoms, provided that greater than 55% of the ester groups of (1) are derived from (IIa), and
(2) at least one carboxylic ester of (III) dipentaerythritol or tripentaerythritol, and (IV) a combination of (a) a monocarboxylic acylating agent having four or five carbon atoms and (b) a branched aliphatic monocarboxylic acylating agent having from 7 to 15 carbon atoms, provided that greater than 55% of the ester groups of (2) are derived from (IVb).

According to a further aspect of the present invention there is provided a liquid composition comprising (A) at least one fluorine-containing hydrocarbon containing one to three carbon atoms; and a lubricant comprising (B) an ester blend of
(1) at least one carboxylic ester of (I) a neo hydroxy compound selected from (i) a neo hydroxy compound with one to four hydroxyl groups, and (ii) a mixture of neo hydroxy compounds having an average of less than 3.5 hydroxyl groups per hydroxy compound, and (II) a monocarboxylic acylating agent selected from (i) a monocarboxylic agent having four or five carbon atoms, and (ii) a combination of (a) a monocarboxylic acylating agent having four or five carbon atoms and (b) a monocarboxylic acylating agent having from 7 to 15 carbon atoms, provided that greater than 55% of the ester groups of (1) are derived from a monocarboxylic acylating agent having four or five carbon atoms, and
(2) at least one carboxylic ester of (III) a neo hydroxy compound selected from (iii) a neo hydroxy compound having greater than four hydroxyl groups per hydroxy compound, and (iv) a mixture of neo hydroxy compounds having an average of greater than 4.5 hydroxyl groups per hydroxy compound, and (IV) a combination of (a) a monocarboxylic acylating agent having four or five carbon atoms and (b) a monocarboxylic acylating agent having from 7 to 15 carbon atoms, provided that greater than 55% of the ester groups of (2) are derived from (IVb); and
(C) at least one additive selected from an alkyl phosphite, an alkyl phosphonic acid ester, a nitrogen containing heterocycle, and a mixture thereof.

Methods of lubricating refrigeration systems are also described. The liquid compositions are useful particularly as refrigeration liquids in refrigerators and air-conditioners including automotive, home and industrial air-conditioners.

Various preferred features and embodiments of the present invention will now be described by way of non-limiting example.

Throughout this specification and claims, all parts and percentages are by weight, temperatures are in degrees Celsius, and pressures are at or near atmospheric pressure unless otherwise clearly indicated.

As used in this specification and in the appended claims, the terms "hydrocarbyl" and "hydrocarbylene" denote a group having a carbon atom directly attached to the polar group and having a hydrocarbon or predominantly hydrocarbon character within the context of this invention. Such groups include the following:
(1) Hydrocarbon groups; that is, aliphatic, (e.g., alkyl or alkenyl), alicyclic (e.g., cycloalkyl or cycloalkenyl), and the like, as well as cyclic groups wherein the ring is completed through another portion of the molecule (that is, any two indicated substituents may together form an alicyclic group). Such groups are known to those skilled in the art. Examples include methyl, ethyl, octyl, decyl, octadecyl, cyclohexyl, etc.
(2) Substituted hydrocarbon groups; that is, groups containing non-hydrocarbon substituents which, in the context of this invention, do not alter the predominantly hydrocarbon character of the group. Those skilled in the art will be aware of suitable substituents. Examples include halo, hydroxy, alkoxy, etc.
(3) Hetero groups; that is, groups which, while predominantly hydrocarbon in character within the context of this invention, contain atoms other than carbon in a chain or ring otherwise composed of carbon atoms. Suitable hetero atoms will be apparent to those skilled in the art and include, for example, nitrogen, oxygen and sulfur.

In general, no more than about three substituents or hetero atoms, and preferably no more than one, will be present for each 10 carbon atoms in the hydrocarbyl group.

Terms such as "alkyl", "alkylene", etc. have meanings analogous to the above with respect to hydrocarbyl and hydrocarbylene.

The term "hydrocarbon-based" also has the same meaning and can be used interchangeably with the term hydrocarbyl when referring to molecular groups having a carbon atom attached directly to the polar group.

The term "lower" as used herein in conjunction with terms such as hydrocarbyl, hydrocarbylene, alkylene, alkyl, alkenyl, alkoxy, and the like, is intended to describe such groups which contain a total of up to 7 carbon atoms, per se and includes methyl, ethyl, propyl, butyl, pentyl, hexyl and heptyl groups.

Viscosity, unless otherwise indicated, is kinematic viscosity and is measured by ASTM D-2270.

For purpose of this invention, equivalent weight of polyol is determined by dividing the formula weight of the polyol by the number of hydroxyl groups. Equivalents of polyol is determined by dividing the amount of the polyol by its equivalent weight. For polycarboxylic acylating agents or anhydrides, the equivalent weight is determined by dividing the formula weight of the acylating agent or anhydride by the number of carboxylic groups which form esters. For example, an anhydride contributes two carboxyl groups which can form ester. Therefore, the equivalent weight of anhydride, such as succinic anhydride, would be the formula weight of the anhydride divided by the number of carboxyl group. For succinic anhydride, the number is two. Of course, those of skill in the art will appreciate that an excess of acylating agent, i.e., more than a single equivalent, may be used.

When a compound or component is indicated herein as being "soluble", the compound or component is soluble in the liquid compositions of the invention comprising the fluorine-containing hydrocarbon and the lubricant. For example, a compound or component is considered "soluble" so long as it is soluble in the liquid compositions, even though it may be insoluble in the fluorine-containing hydrocarbon per se.

The term "consisting essentially of" refers to compositions that include the ingredients listed in the claim as well as other ingredients that do not materially affect the basic and novel characteristics of the liquid compositions.

### (A) Fluorine-Containing Hydrocarbon.

The liquid compositions of the present invention comprise at least one fluorine-containing hydrocarbon. That is, the fluorine-containing hydrocarbons contain at least one C-H bond as well as C-F bonds.In addition to these two essential types of bonds, the hydrocarbon also may contain other carbon-halogen bonds such as C-Cl bonds. Because the liquid compositions of the present invention are primarily intended for use as refrigerants, the fluorine-containing hydrocarbon preferably contains one to three, or to two carbon atoms, and more preferably two carbon atoms.

As noted above, the fluorine-containing hydrocarbons useful in the liquid compositions of the present invention may contain other halogens such as chlorine. However, in one preferred embodiment, the hydrocarbon contains only carbon, hydrogen and fluorine. These compounds containing only carbon, hydrogen and fluorine are referred to herein as fluorohydrocarbons or hydrofluorocarbons. The hydrocarbons containing chlorine as well as fluorine and hydrogen are referred to as chlorofluorohydrocarbons or hydrochlorofluorocarbons.

Specific examples of the fluorine-containing hydrocarbons useful in the liquid compositions of the present invention include HCFC-22 (CHClF₂), HCFC-123 (CHCl₂CF₃), HCFC-141b (CH₃CCl₂F), HFC-134a (CH₂FCF₃).

Examples of other fluorine-containing hydrocarbons which may be useful in the liquid compositions of the present invention include trifluoromethane (HFC-23), 1,1,1-trifluoroethane (HFC-143a), 1,1-difluoroethane (HFC-152a), 2-chloro-1,1,1,2-tetrafluoroethane (HCFC-124), 1-chloro-1,1,2,2-tetrafluoroethane (HCFC-124a), 1-chloro-1,1-difluoroethane (HCFC-142b), and 1,1,2,2-tetrafluoroethane (HFC-134). Other refrigerants such as perfluoropropane (HFC-218), perfluorocyclopropane (HFC-216), perfluoropropylene oxide, 1,3-perfluoro propylene oxide and pentafluorodimethyl ether may be used with the lubricant. In the refrigerant art, the fluorohydrocarbons are often identified merely with the prefix "R" in place of the above letters.For example HFC-23 is R-23, HCFC-124 is R-124, etc.

In general, fluorine-containing hydrocarbons which are useful as refrigerants are fluoromethanes and fluoroethanes boiling at a relatively low temperature at atmospheric pressure, e.g., below 30°C. Mixtures of fluorine-containing hydrocarbons may be used, and the amount of each fluorohydrocarbon in the mixture may be varied as desired. Examples of fluorohydrocarbon mixtures useful as (A) include: 142(b)/22; 134(a)/23; 22/124/152(a), etc. The useful fluorocarbon refrigerants serve to transfer heat in a refrigeration system by evaporating and absorbing heat at a low temperature and pressure, e.g., near ambient temperature and atmospheric pressure, and by releasing heat on condensing at a higher temperature and pressure.

The amount of fluorine-containing hydrocarbon is the level typically used for the refrigeration system. The liquid compositions of the present invention generally contain from about 10%, or about 20% up to about 90%, or to about 85% of the fluorine-containing hydrocarbon. In one embodiment, the fluorine-containing hydrocarbon is present in an amount from about 45%, or about 50%, or about 55% up to about 90%, or to about 80%, or to about 75% by weight of the liquid composition. More generally, the liquid compositions will comprise from about 50% to about 99% by weight of the fluorine-containing hydrocarbon. In another embodiment, the liquid compositions contain from about 70% to about 99% by weight of the fluorine-containing hydrocarbon. When the fluorine-containing hydrocarbon is used at levels greater than 50% by weight of the lubricant, then the liquid compositions are generally suited for use as automotive and commercial and industrial refrigeration systems.

In one embodiment, the fluorine-containing hydrocarbon is present in an amount from about 10%, or about 25%, or about 30% up to about 55%, or to about 50%, or to about 45% by weight of the lubricant. When the fluorine-containing hydrocarbon is present in an amount less than about 45%, then the liquid compositions are generally suited for household refrigeration systems.

### (B) Carboxylic Esters

The carboxylic ester blend used in the liquid composition of the present invention are prepared by reacting specific carboxylic acylating agents and specific neo hydroxy compounds. The blend consists of carboxylic esters (1) and (2).

Monocarboxylic ester (1) is prepared from (I) a neo hydroxy compound selected from the group consisting of (i) a neo hydroxy compound with one to four hydroxyl groups, and (ii) a mixture of neo hydroxy compounds having an average of less than 3.5 hydroxyl groups per hydroxy compound, and (II) a monocarboxylic acylating agent selected from the group consisting of (i) a monocarboxylic acylating agent having four or five carbon atoms, and (ii) a combination of (a) a monocarboxylic acylating agent having four or five carbon atoms and (b) a monocarboxylic acylating agent having from about 7 to about 15 carbon atoms, provided that greater than 55%, or greater than 57%, or greater than 59% of the ester groups of (1) are derived from the monocarboxylic acylating agent having four or five carbon atoms.

Esters may be prepared from any hydroxy compound, such as a polyhydroxy compound. These hydroxy compounds include glycols and triols, such as butanediol, hexanediol, glycerol, butanetriol, and hexanetriol. However, the inventor has discovered that esters derived from neo alcohols have beneficial solubility in fluorine-containing hydrocarbon, and thermal and chemical stability. The neo hydroxy compound (I) is (i) neo hydroxy compounds having two or three hydroxyl groups, preferably three hydroxyl groups. Examples of neo hydroxy compound (i) include neopentyl glycol and a trimethylolalkanes, such as trimethylolmethane, trimethylolethane, trimethylolpropane and trimethylolbutane. A preferred neo hydroxy compound is trimethylolpropane.

In another embodiment, the neo hydroxy compound (I) is (ii) a mixture of neo hydroxy compounds having an average of 3.5 hydroxyl groups per hydroxy compound. The average number of hydroxyl groups per hydroxy compound is determined by dividing the total number of hydroxyl groups in the mixture by the total number of hydroxy compounds in the mixture. For instance, a (50:50) molar mixture of trimethylolpropane and neopentyl glycol has 2.5 hydroxyl groups per hydroxy compound. A (75:25) molar mixture of trimethylolbutane and neopentylglycol has an average of 2.75 hydroxyl groups per hydroxy compound. Any mixture of hydroxyl compound may be used provided that the resultant esters were soluble in the liquid compositions of the present invention. In one embodiment, the mixture is prepared from neo hydroxy compounds having from two to about four, preferably to about three hydroxyl groups per hydroxy compound. Examples of neo hydroxy compound which may be used to form the mixture (ii) include pentaerythritol, neopentylglycol, and trimethylolalkanes, as described above. Example of molar mixtures of hydroxy compound includes trimethylolpropane and pentaerythritol (80:20), trimethylolbutane and neopentyl glycol (50:50), and trimethylolpropane and neopentyl glycol (75:25).

The above hydroxy compounds are reacted with (II) monocarboxylic acylating agents selected from (i) monocarboxylic acylating agents having four or five carbon atoms, and (ii) a combination of (a) a monocarboxylic acylating agent having four or five carbon atoms and (b) a monocarboxylic acylating agent having from about 7 to about 15 carbon atoms, provided greater than 55%, or greater than 57%, or greater than 59% of the ester groups of (1) are derived from monocarboxylic acylating agents having four or five carbon atoms. The monocarboxylic acylating agents (II) are preferably aliphatic acylating agents. In one embodiment, monocarboxylic acylating agent is linear or branched. In one embodiment monocarboxylic acylating agent (II) may be isobutyric, valeric, 2-methylbutyric, or neopentanoic acid or anhydride. Commercially available isopentanoic acid is a mixture of linear pentanoic acid (valeric acid) and methyl-butanoic acid with approximate weight ratio of (66:34). In another embodiment, the monocarboxylic acylating agent may be neo pentanoic acid, available from Exxon Chemical Company.

The monocarboxylic acylating agent (IIb) generally contains from about 7, or about 8 up to about 15, or to about 12, or to about 10 carbon atoms. This acylating agent may be linear or branched, including "iso" and "neo" branched acylating agents. Examples of these acylating agents include n-octanoic acid, neoheptanoic acid, neodecanoic acid, 2,2,4-trimethylpentanoic acid, 2-hexyldecanoic acid, isostearic acid, 2-methylhexanoic acid, 3,5,5-trimethylhexanoic acid, 2-ethylhexanoic acid, isooctanoic acid, isononanoic acid, isoheptanoic acid, isodecanoic acid, neoheptanoic acid, neodecanoic acid, and ISO Acids and NEO Acids available from Exxon Chemical Company, Houston, Texas USA. ISO Acids are isomer mixtures of branched acids and include commercial mixtures such as ISO Heptanoic Acid, ISO Octanoic Acid, and ISO Nonanoic Acid, as well as developmental products such as ISO Decanoic Acids and ISO 810 Acid. Of the ISO Acids, ISO Octanoic acid and ISO Nonanoic acid are preferred. Neo acids include commercially available mixtures such as NEO Pentanoic Acid, NEO Heptanoic Acid, and NEO Decanoic Acid, as well as developmental products such as ECR-909 (NEO C₉) Acid, and ECR-903 (NEO C₁₂₁₄) Acid and commercial mixtures of branched chain carboxylic acids such as the mixture identified as NEO 1214 acid from Exxon.

The carboxylic ester blends also include (2) at least one carboxylic ester of (III) a neo hydroxy compound selected from the group consisting of (iii) a neo hydroxy compound having greater than four hydroxyl groups per hydroxy compound, and (iv) a mixture of neo hydroxy compounds having an average greater than 4.5 hydroxyl groups per hydroxy compound, and (IV) a combination of (a) a monocarboxylic acylating agent having four or five carbon atoms and (b) an monocarboxylic acylating agent having from about 7 to about 15 carbon atoms, provided that greater than 55%, or greater than 57 %, or greater than 59 % of the ester groups of (2) are derived from (IVb). The carboxylic acylating agents are described above.

The neo hydroxy compound (iii) has more than four hydroxyl groups per hydroxy compound. These neo hydroxy compounds generally contain from about five, or about six up to about 12, or to about 10, or to about 8 hydroxyl groups per hydroxy compound. Examples of these compounds include dipentaerythritol, tripentaerythritol, and di-trimethylolpropane

In another embodiment, the neo hydroxy compound (III) is (iv) a mixture of neo hydroxy compounds having an average of greater than 4.5 hydroxyl groups per hydroxy compound. The determination of this average is the same as described above for (Iii). Examples of (iv) include dipentaerythritol and tripentaerythritol (75:25), trimethylolpropane and dipentaerythritol (80:20), and tripentaerythritol and pentaerythritol (75:25).

The carboxylic esters of the liquid compositions are prepared by reacting at least one carboxylic acylating agent with at least one hydroxy compound. The formation of esters by the interaction of carboxylic acylating agents and alcohols is acid catalyzed and is a reversible process which can be made to proceed to completion by use of a large amount of alcohol or carboxylic acylating agent, or by removal of the water as it is formed in the reaction. If the ester is formed by transesterification of a lower molecular weight carboxylic ester, the reaction can be forced to completion by removal of the low molecular weight alcohol or acid formed as a result of a transesterification reaction. The esterification reaction can be catalyzed by either organic acids or inorganic acids. Examples of inorganic acids include sulfuric acids and acidified clays. A variety of organic acids can be utilized including paratoluenesulfonic acid, and acidic resins, such as Amberlyst 15, etc. Organometallic catalysts include, for example, tetraisopropoxy orthotitanate.

The following examples relate to the esters used in making the blends as described herein. Unless otherwise indicated, in the following examples, as well as in the rest of the specification and claims, parts and percentages are by weight, the pressure is atmospheric pressure and the temperature is in degrees Celsius. Examples 1-5 relate to ester (1) of the blend and Examples 6-9 relate to esters (2) of the blend.

### Example 1

A reaction vessel is charged with 2145 parts (16 moles) of trimethylolpropane and 2305 parts (16 moles) of 2,2,4-trimethylpentanoic acid.The mixture is heated to 150°C and the temperature is maintained for 2-1/2 hours. Distillate (260 ml.) is collected. The reaction mixture is cooled to 145°C where 3098 parts (35.2 moles) of isobutyric acid is added to the reaction vessel over 1-1/2 hours. The reaction mixture cools to 100°C after the addition of the isobutyric acid. SE-100 aromatic solvent, commercially available from Ohio Solvents (100 ml.), is added to the reaction vessel. The reaction mixture is heated to 145°C and the temperature is maintained for 17 hours. The reaction mixture is vacuum stripped at 145°C, 20 mm Hg for 18 hours. The neutralization acid number of the residue after vacuum stripping is 0.15. The reaction mixture is vacuum stripped for an additional 4 hours and the residue is filtered through a mixture of diatomaceous earth and aluminum oxide. The filtrate has a kinematic viscosity of 13.82 at 40°C and 3.1 at 100°C. The residue has a 0.03 total acid number.

### Example 2

A reaction vessel is charged with 3082 (69 equivalents) of trimethylolpropane, 3312 (23 equivalents) of 2,2,4-trimethylpentanoic acid, 3634 (46 equivalents) of isobutyric anhydride and 10 parts (0.1 equivalent) of methanesulfonic acid. The reaction mixture is heated under nitrogen to 115°C. The temperature is maintained between 115°C to 150°C for 6 hours. The reaction temperature is maintained at 150°C for 10 hours while 798 milliliters of distillate is collected. The mixture is heated to 200°C and held for 8 hours while 32 milliliters of distillate is collected. The total distillate collected is 860 milliliters. The mixture is cooled to 130°C where 350 parts (4.4 equivalents) of isobutyric anhydride are added to the reaction vessel. The mixture is stirred at 130°C for 24 hours. The reaction mixture is vacuum stripped at 8-10 mm Hg and 120-150°C for 8 hours. The acid number after the stripping was 4.2. The reaction mixture is stripped to 190°C and 8-10 mm Hg for 6 hours. The acid number after this stage of stripping is 0.84. The residue is vacuum stripped for 8 hours at 195°C and 10 mm Hg.The acid number after this stage of stripping is 0.051. The residue of the product has a kinematic viscosity of 14.0 centistokes at 40°C and 3.1 centistokes at 100°C. The acid number of the product is 0.05.

### Example 3

A reaction vessel is charged with 1139 parts (8.5 moles) of trimethylolpropane, 1224 parts (8.5 moles) of 2,2,4-trimethylpentanoic acid, and 6 parts of methanesulfonic acid. The mixture is heated under nitrogen to 117°C and the temperature is maintained for 3 hours while a total of 90 milliliters of water is collected. The mixture is then heated to 150°C and the temperature is maintained for 5 hours. The total water collected was 153 milliliters. Isobutyric acid (1496 parts, 17 moles) is added to the reaction mixture. The mixture is heated to 120°C and a temperature is maintained between 120°C and 125°C for 19 hours. The acid number of the reaction mixture is 41. The reaction mixture is heated to 145°C and the temperature is maintained for 22 hours. The acid number of the reaction mixture is 24. An additional charge is isobutyric acid (200 parts, 2.27 moles) is added to the reaction mixture. The mixture is heated to 145°C and the temperature is maintained for 22 hours. The reaction mixture is vacuum stripped to 125°C and 20-25 mm Hg for 3 hours. The residue is then subjected to additional vacuum stripping at 150°C and 10 mm Hg for 3 hours. The reaction mixture is then vacuum stripped to 165-l75°C and 8-10 mm Hg for 5 hours. The neutralization acid number 2-phenolphthalein of the residue is 0.29. The residue is vacuum stripped at 175°C and 8-10 mm Hg for 6 hours. After this vacuum stripping, the neutralization acid number is 0.16. The residue is filtered through a 3/4" pad of aluminum oxide. The filtrate has a acid number of 0.11 and a kinematic viscosity of 14.67 (at 40°C) and 3.23 (at 100°C).

### Example 4

A reaction vessel is charged with 660 parts (5.5 moles) of trimethylolethane, 1122 parts (11.0 moles) of neopentanoic acid, 869 parts (5.5 moles) of 3,5,5-trimethylhexanoic acid, and 3 parts of tetraisopropoxy titanate. The mixture was heated to 220°C for 72 hours while water is removed. The reaction mixture is stripped for 8 hours at 180-200°C. The residue is treated with 200 parts of alumina and filtered through cloth and diatomaceous earth. The filtrate is the desired product and has a kinematic viscosity of 23.6 centistokes (at 40°C) and 4.0 centistokes (at 100°C). The filtrate has a acid number of 0.08.

### Example 5

A reaction vessel is charged with 603 parts (4.5 moles) of trimethylolpropane, 441 parts (3 moles) of 2,2,4-trimethylpentanoic acid, 1065 parts of isopentanoic acid (a mixture of valeric acid and 2-methylbutyric acid having a weight ratio of (66:34) and available commercially from Union Carbide), and 4 parts of methanesulfonic acid. The reaction mixture is heated to 150°C and the temperature is maintained for six hours. Water is collected azeotropically (220 milliliters). The reaction temperature is maintained for an additional 31 hours where an additional 10 milliliters of water is collected. The reaction mixture is vacuum stripped to 150°C and 20 mm Hg for 18 hours. The neutralization acid number of the residue is 0.12. The residue is filtered through alumina and diatomaceous earth. The filtrate is the desired product.

### Example 6

A reaction vessel is charged with 2286 parts (9.0 moles) of dipentaerythritol, 1422 parts (9 moles) of 3,5,5-trimethylhexanoic acid, 1836 parts (18 moles) of neopentanoic acid, and 8 parts of methanesulfonic acid. The reaction is heated to 140°C and the temperature is maintained for 18 hours. Water is collected (460 milliliters) by distillation. An additional charge of 3,5,5-trimethylhexanoic acid (4266 parts, 27 moles) is added to the reaction vessel. The reaction is heated to 200°C and the temperature is maintained for 18 hours. The reaction is vacuum stripped to 200°C and 10 mm Hg. After 15 hours of stripping the acid number was 0.2. The residue was treated with alumina (800 parts) and the mixture is heated to 110°C and the temperature is maintained for 4 hours. The product is filtered and the filtrate is the desired product. The filtrate has a kinematic viscosity of 720 centistokes at 40°C and 30.5 centistokes (at 100°C).

### Example 7

A reaction vessel is charged with 166 parts (1.22 moles) of pentaerythritol, 151 parts (0.4 moles) of tripentaerythritol, 439 parts (2.7 moles) of 3,5,5-trimethylhexanoic acid, 391 parts (2.7 moles) of 2,2,4-trimethylpentanoic acid, 214 parts (1.35 moles) of isobutyric anhydride, and 4 parts of tetra-isopropoxy titanate. The reaction mixture is heated to 170°C and the temperature is maintained for 24 hours. Water is collected by distillation (130 milliliters). The reaction is vacuum stripped for 3 hours at 170°C and 1 mm Hg. The product is filtered and the residue has a kinematic viscosity of 133.8 centistokes (at 40°C) and 13.27 centistokes (at 100°C). The acid number of the product is 0.1.

### Example 8

A reaction vessel is charged with 192 parts (1.41 moles) of pentaerythritol, 242 parts (0.95 moles) of dipentaerythritol, 1337 parts (8.46 moles) of 3,5,5-trimethylhexanoic acid, 406 parts (2.82 moles) of 2,2,4-trimethylpentanoic acid, and 3 parts of tetraisopropoxy titanate. The reaction mixture is heated to 210°C and the temperature is maintained for 30 hours while water is removed. The reaction mixture is vacuum stripped to 210°C and 10 mm Hg. The residue is treated with alumina and filtered. The filtrate is the desired product and has a kinematic viscosity of 171.7 centistokes (at 40°C) and 15.4 centistokes (at 100°C).

### Example 9

A reaction vessel is charged with 374 parts (2.75 moles) of pentaerythritol, 465 parts (1.83 moles) of dipentaerythritol, 3042 parts (19.25 moles) of 3,5,5-trimethylhexanoic acid, 217 parts (1.37 moles) of isobutyric anhydride, and 4 parts of methanesulfonic acid. The reaction mixture is heated to 130°C and the temperature is maintained for 24 hours, while 360 milliliters of water is collected. An additional charge of isobutyric anhydride (20 grams) is added to the reaction mixture at 100°C. The reaction mixture is heated to 190°C and blown with nitrogen at 2 standard cubic feet per hour for 6 hours. The product is vacuum stripped for 24 hours at 200°C and 10 mm Hg. After 16 hours of vacuum stripping the acid number is 0.15. After 24 hours of vacuum stripping the acid number is 0.1. The residue is treated with alumina (300 parts) at 120°C. The acid number after alumina treatment is 0.05. The mixture is filtered through a pad of alumina and diatomaceous earth. The filtrate is the desired product and has a kinematic viscosity of 182 centistokes (at 40°C) and 15.6 centistokes (at 100°C). The filtrate has a acid number of 0.05.

In one embodiment, the esters (B) contain branched alkyl groups and generally are free of acetylenic and aromatic unsaturation. The soluble lubricants of this invention also are preferably free of olefinic unsaturation except that some olefinic unsaturation may be present so long as the lubricant is soluble.

The carboxylic esters components or the lubricant (B) are soluble in the fluorine-containing hydrocarbons and, in particular, in the fluorohydrocarbons such as 1,1,1,2-tetrafluoroethane. The lubricant components are soluble over a wide temperature range and, in particular, at low temperatures. The solubility of the components in fluorohydrocarbons such as 1,1,1,2-tetrafluoroethane at low temperatures is determined in the following manner. The component (0.5 gram) is placed in a thick-walled glass vessel equipped with a removable pressure gauge. The tetrafluoroethane (4.5 grams) is condensed into the cooled (-40°C) glass vessel, and the contents are warmed to the desired temperature and mixed to determine if the lubricant component is soluble in the tetrafluoroethane. If soluble, the temperature of the mixture is reduced until a separation and/or precipitate is observed. The results of this solubility test conducted with several examples of the carboxylic esters used in the lubricants of the present invention are summarised in the following Table I.

**TABLE I**

| Liquid Containing Product of Example | Solubility °C (ppt.) |
|---|---|
| 1 | < -78 |
| 2 | -31 |
| 3 | < -78 |
| 4 | < -40 |
| 6 | -35 |
| 7 | -5 |
| 8 | -24 |

The liquid compositions of the present invention use a blend of esters (1) and (2)The blends are prepared by means known to those in the art. The amount of each ester present is determined by the desired viscosity of the blend. The following table contains blends of esters which may be used in the present invention.

**Table II**

| Blend | Product of | Weight Percent |
|---|---|---|
| A | Example 1 | 40 |
| | Example 6 | 60 |
| B | Example 1 | 60 |
| | Example 6 | 40 |

The liquid compositions may additionally contain (C) at least one additive selected from the group consisting of an alkyl phosphite, an alkyl phosphonic acid ester, a nitrogen-containing heterocycle, and a mixture thereof. The phosphite and/or alkyl phosphonic acid ester is present in an amount sufficient to provide antiwear and/or extreme pressure properties to the lubricant and liquid composition. The phosphite and/or alkyl phosphonic acid ester is present in an amount to provide 0.001%, or to 0.015%, or about 0.025%, to about 1%, or to about 0.5%, or to about 0.2% by weight phosphorus to the lubricant. The nitrogen-containing heterocycle is present in an amount from about 0.001%, or about 0.02%, or about 0.03% up to about 5%, or to about 2%, or to about 1%, or to about 0.5% by weight of the lubricant.

The phosphite and/or the alkyl phosphonic acid ester provide beneficial antiwear and extreme pressure properties to the liquid compositions. The phosphite may be a dialkyl or trialkyl phosphite, preferably a dialkyl phosphite. The alkyl phosphonic acid ester may be an alkyl phosphonic acid diester, preferably a dialkylester. The alkyl groups of the phosphite and the phosphonic acid ester independently contain from 1, or about 3 to about 20, or to about 18, or to about 8 carbon atoms. In one embodiment, the phosphite and the phosphonic acid ester have alkyl groups independently containing from about 3 to about 6, or to about 5 carbon atoms. A number of dialkyl phosphites are commercially available, such as lower dialkyl phosphites, which are preferred. Lower dialkyl phosphites include dimethyl, diethyl, dipropyl, dibutyl, dipentyl and dihexyl phosphites. Phosphites and their preparation are known and many phosphites are available commercially. Also mixed alkyl phosphites, made from a mixture of alcohols, are useful in the present invention. Examples of mixtures of alcohols include ethyl and butyl alcohol; propyl and pentyl alcohol; and methyl and pentyl alcohol. A particularly useful phosphite is dibutyl phosphite.

Alkyl phosphonic acid esters are prepared by means known to those in the art. For example, alkyl phosphonic acid esters may be prepared by reacting an alkyl halide with a trialkyl phosphite. Examples of alkyl phosphonic acid esters include diethyl, butylphosphonate; dibutyl,butylphosphonate; 2-ethylhexyl,2-ethylhexylphosphonate, etc.

The lubricant may additionally contain a nitrogen-containing heterocycle, such as dimercaptothiadiazoles, triazoles, amino-mercaptothiadiazoles, imidazoles, thiazoles, tetrazoles, hydroxyquinolines, oxazolines, imidazolines, thiophenes, indoles, indazoles, quinolines, benzoxazines, dithiols, oxazoles, oxatriazoles, pyridines, piperazines, triazines, and derivatives of any one or more thereof. In one embodiment, the nitrogen containing heterocycle is a triazole or derivative thereof, a thiazole or derivative thereof, a mercaptothiazole or derivative thereof and a thiadiazole or derivative thereof, preferably a triazole or derivative thereof. These additives provide metal deactivating, metal passivating and corrosion controlling character to the liquid compositions. Examples of useful metal deactivators include dimercaptothiadiazoles and derivatives thereof, substituted and unsubstituted triazoles (e.g., benzotriazole, tolyltriazole, octylbenzotriazole, and the like), mercaptobenzothiazoles, etc. Examples of these compounds are benzotriazole, alkyl-substituted benzotriazole (e.g., tolyltriazole, ethylbenzotriazole, hexylbenzotriazole, octylbenzotriazole, etc.), aryl-substituted benzotriazole (e.g., phenol benzotriazoles, etc.), and alkylaryl- or arylalkyl-substituted benzotriazole and substituted benzotriazoles where the substituent may be hydroxy, alkoxy, halo (especially chloro), nitro, carboxy and carboxyalkoxy. Preferably, the triazole is a benzotriazole or an alkylbenzotriazole in which the alkyl group contains 1 to about 20 carbon atoms, preferably 1 to about 8 carbon atoms.

The nitrogen containing heterocycle (C) may also be the reaction product of at least one of the above triazoles, at least one amine and an aldehyde or aldehyde precursor. The triazole is preferably a benzotriazole. The amine can be one or more mono- or polyamines. These monoamines and polyamines can be primary amines, secondary amines or tertiary amines. Examples of polyamines include polyalkylenepolyamines, and heterocyclic polyamines. Polyalkylenepolyamines include polyethylenepolyamines, such as diethylenetriamine, triethylenetriamine, tetraethylenepentaamine, etc.

The aldehyde is typically a hydrocarbon-based aldehyde, preferably a lower aliphatic aldehyde. Suitable aldehydes include formaldehyde, benzaldehyde, acetalde-hyde, the butyraldehydes, hydroxybutyraldehydes and heptanals, as well as aldehyde precursors which react as aldehydes under the conditions of the reaction such as paraformaldehyde, paraldehyde, formalin and methanal. Formaldehyde and its precursors (e.g., paraformaldehyde, trioxane) are preferred. Mixtures of aldehydes may be used.

An example of a useful triazole derivative is Reomet® 39. This material is a triazole derivative available commercially from Ciba-Geigy Corporation.

The liquid compositions of the present invention are characterized as having improved thermal and chemical stability over a wide temperature range. The liquid compositions with (C) phosphites, phosphonates, and/or nitrogen-containing heterocycles have improved antiwear and corrosion stability properties. The liquid compositions have beneficial viscosity properties. Preferably the liquid compositions have a viscosity of 5-400 centistokes (cSt) measured at 40°C

Other additives, if soluble in the liquid, known to be useful for improving the properties of halogen-containing hydrocarbon refrigerants can be included in the liquid compositions of the present invention to improve the characteristics of the liquid as a refrigerant. However, hydrocarbon oils such as mineral oil generally are not included in and are most often excluded from the liquid compositions of the invention, particularly when the fluorine-containing hydrocarbon contains no other halogens. Hydrocarbon lubricants, however, may be present if the liquid compositions are used to retrofit a compressor system which had previously used a hydrocarbon lubricant.

Other additives may be included in the liquid compositions of the present invention to enhance the performance of the liquids include extreme-pressure and antiwear agents, oxidation and thermal-stability improvers, corrosion-inhibitors, viscosity-index improvers, pour point and/or floc point depressants, detergents, dispersants, anti-foaming agents, viscosity adjusters, metal deactivators, etc. As noted above, these supplementary additives must be soluble in the liquid compositions of the invention. Included among the materials which may be used as extreme-pressure and anti-wear agents are phosphates, phosphate esters, thiophosphates such as zinc diorganodithiophosphates, chlorinated waxes, sulfurized fats and olefins, organic lead compounds, fatty acids, molybdenum complexes, borates, halogen-substituted phosphorous compounds, sulfurized Diels Alder adducts, organic sulfides, metal salts of organic acids, etc. Sterically hindered phenols, aromatic amines, dithiophosphates, sulfides and metal salts of dithioacids are useful examples of oxidation and thermal stability improvers. Compounds useful as corrosion-inhibitors include organic acids, organic amines, organic phosphates, organic alcohols, metal sulfonates, etc. VI improvers include polyolefins such as polyesterbutene, polymethacrylate, polyalkylstyrenes, etc. Pour point and floc point depressants include polymethacrylates, ethylene-vinyl acetate copolymers, succinamic acid-olefin copolymers, ethylene-alpha olefin copolymers, etc. Detergents include sulfonates, long-chain alkyl-substituted aromatic sulfonic acids, phenylates, metal salts of alkylphenols, alkylphenol-aldehyde condensation products, metal salts of substituted salicylates, etc. Silicone polymers are a well known type of anti- foam agent. Viscosity adjusters are exemplified by polyisobutylene, polymethacrylates, polyalkylstyrenes, naphthenic oils, alkyl benzene oils, polyesters, polyvinyl chloride, polyphosphates, etc.

The following example (TABLE 1) relates to formulations which are useful as organic lubricant (B) in the present invention.

**TABLE 1**

| | |
|---|---|
| Product of Ex. A | 99.3 |
| DBPH¹ | 0.5 |
| Tolyltriazole | 0.1 |
| Oleyamide | 0.1 |

| | |
|---|---|
| **(1) Dibutyl phosphite** | |

The liquid compositions of the present invention are particularly useful as refrigerants in various refrigeration systems which are compression-type systems such as refrigerators, freezers, and air-conditioners including automotive, home and industrial air-conditioners. The following examples are illustrative of the liquid compositions of the present invention.

| | Parts by Wt. |
|---|---|
| Example A | |
| 1,1,1,2-tetrafluoroethane (HCFC-134a) | 60 |
| Blend A | 40 |

| Example B | |
|---|---|
| HCFC-134a | 80 |
| Blend B | 20 |

While the invention has been explained in relation to its preferred embodiments, it is to be understood that various modifications thereof will become apparent to those skilled in the art upon reading the specification. Therefore, it is to be understood that the invention disclosed herein is intended to cover such modifications as fall within the scope of the appended claims.

## Claims (Claims for the following Contracting State(s): AT, BE, CH, DE, DK, FR, GB, GR, IT, LI, LU, NL, SE, MC, PT, IE)

1. A liquid composition comprising:
(A) at least one fluorine-containing hydrocarbon containing one to three carbon atoms; and a lubricant comprising (B) a blend of
(1) at least one carboxylic ester of (I) a neo hydroxy compound selected from (i) a neo hydroxy compound with one to four hydroxyl groups, and (ii) a mixture of neo hydroxy compounds having an average of less than 3.5 hydroxyl groups per hydroxy compound, and (II) a monocarboxylic acylating agent selected from (i) a monocarboxylic acylating agent having four or five carbon atoms, and (ii) a combination of (a) a monocarboxylic acylating agent having four or five carbon atoms and (b) a monocarboxylic acylating agent having from 7 to 15 carbon atoms, provided that greater than 55% of the ester groups of (1) are derived from a monocarboxylic acylating agent having four or five carbon atoms, and
(2) at least one carboxylic ester of (III) a neo hydroxy compound selected from (iii) a neo hydroxy compound having greater than four hydroxyl groups per hydroxy compound, and (iv) a mixture of neo hydroxy compounds having an average greater than 4.5 hydroxyl groups per hydroxy compound, and (IV) a combination of (a) a monocarboxylic acylating agent having four or five carbon atoms and (b) a monocarboxylic acylating agent having from 7 to 15 carbon atoms, provided that greater than 55% of the ester groups of (2) are derived from (IVb).

2. A liquid composition of claim 1 wherein fluorine is the only halogen in the fluorine-containing hydrocarbon (A).

3. A liquid composition of either of claims 1 and 2 wherein the fluorine-containing hydrocarbon is 1,1,1,2-tetrafluoroethane.

4. A liquid composition of any preceding claim wherein the neo hydroxy compound (I) is a trimethylolalkane.

5. A liquid composition of any preceding claim wherein the neo hydroxy compound (I) is trimethylolpropane.

6. A liquid composition of any preceding claim wherein the neo hydroxy compound (III) contains an average of 5 or more hydroxyl groups.

7. A liquid composition of any preceding claim wherein the neo hydroxy compound (III) is dipentaerythritol or tripentaerythritol.

8. A liquid composition of any preceding claim wherein the monocarboxylic acylating agent of (IIa) and (IVa) is independently isobutyric, valeric, 2-methylbutyric or neopentanoic acid or anhydride.

9. A liquid composition of any preceding claim wherein the monocarboxylic acylating agent of (IIb) and (IVb) is independently a branched monocarboxylic acylating agent having 8 or 9 carbon atoms.

10. A liquid composition of any preceding claim further comprising (C) at least one additive selected from an alkyl phosphite, an alkyl phosphonic acid ester, a nitrogen containing heterocycle, and a mixture thereof.

11. A liquid composition of claim 10 wherein (C) is an alkyl phosphite or alkyl phosphonic acid ester independently having from 1 to 20 carbon atoms in each alkyl group.

12. A liquid composition of claim 10 wherein (C) is an alkyl phosphite or alkyl phosphonic acid ester independently having from 3 to 8 carbon atoms in each alkyl group.

13. A liquid composition of claim 10 wherein (C) is dibutyl phosphite.

14. A liquid composition of claim 10 wherein (C) is dibutyl, butyl phosphonate.

15. A liquid composition of claim 10 wherein (C) is a triazole or a derivative thereof.

16. A liquid composition of claim 10 wherein (C) is tolyltriazole, benzotriazole, or a reaction product of a benzotriazole, and amine and an aldehyde or aldehyde precursor.

17. A liquid composition of claim 10 wherein (C) is a mixture of an alkyl phosphite independently having from 1 to 20 carbon atoms in each alkyl group, and a triazole or derivative thereof.

18. A method of lubricating a refrigeration system comprising the steps of introducing into the refrigeration system a liquid composition of any one of claims 1 to 17 ; and operating the system.

## Claims (Claims for the following Contracting State(s): ES)

1. A process for the preparation of a liquid composition comprising admixing:
(A) at least one fluorine-containing hydrocarbon containing one to three carbon atoms; and a lubricant comprising (B) a blend of
(1) at least one carboxylic ester of (I) a neo hydroxy compound selected from (i) a neo hydroxy compound with one to four hydroxyl groups, and (ii) a mixture of neo hydroxy compounds having an average of less than 3.5 hydroxyl groups per hydroxy compound, and (II) a monocarboxylic acylating agent selected from (i) a monocarboxylic acylating agent having four or five carbon atoms, and (ii) a combination of (a) a monocarboxylic acylating agent having four or five carbon atoms and (b) a monocarboxylic acylating agent having from 7 to 15 carbon atoms, provided that greater than 55% of the ester groups of (1) are derived from a monocarboxylic acylating agent having four or five carbon atoms, and
(2) at least one carboxylic ester of (III) a neo hydroxy compound selected from (iii) a neo hydroxy compound having greater than four hydroxyl groups per hydroxy compound, and (iv) a mixture of neo hydroxy compounds having an average greater than 4.5 hydroxyl groups per hydroxy compound, and (IV) a combination of (a) a monocarboxylic acylating agent having four or five carbon atoms and (b) a monocarboxylic acylating agent having from 7 to 15 carbon atoms, provided that greater than 55% of the ester groups of (2) are derived from (IVb).

2. A process of claim 1 wherein fluorine is the only halogen in the fluorine-containing hydrocarbon (A).

3. A process of either of claims 1 and 2 wherein the fluorine-containing hydrocarbon is 1,1,1,2-tetrafluoroethane.

4. A process of any preceding claim wherein the neo hydroxy compound (I) is a trimethylolalkane.

5. A process of any preceding claim wherein the neo hydroxy compound (I) is trimethylolpropane.

6. A process of any preceding claim wherein the neo hydroxy compound (III) contains an average of 5 or more hydroxyl groups.

7. A process of any preceding claim wherein the neo hydroxy compound (III) is dipentaerythritol or tripentaerythritol.

8. A process of any preceding claim wherein the monocarboxylic acylating agent of (IIa) and (IVa) is independently isobutyric, valeric, 2-methylbutyric or neopentanoic acid or anhydride.

9. A process of any preceding claim wherein the monocarboxylic acylating agent of (IIb) and (IVb) is independently a branched monocarboxylic acylating agent having 8 or 9 carbon atoms.

10. A process of any preceding claim further comprising (C) at least one additive selected from an alkyl phosphite, an alkyl phosphonic acid ester, a nitrogen containing heterocycle, and a mixture thereof.

11. A process of claim 10 wherein (C) is an alkyl phosphite or alkyl phosphonic acid ester independently having from 1 to 20 carbon atoms in each alkyl group.

12. A process of claim 10 wherein (C) is an alkyl phosphite or alkyl phosphonic acid ester independently having from 3 to 8 carbon atoms in each alkyl group.

13. A process of claim 10 wherein (C) is dibutyl phosphite.

14. A process of claim 10 wherein (C) is dibutyl, butyl phosphonate.

15. A process of claim 10 wherein (C) is a triazole or a derivative thereof.

16. A process of claim 10 wherein (C) is tolyltriazole, benzotriazole, or a reaction product of a benzotriazole, and amine and an aldehyde or aldehyde precursor.

17. A process of claim 10 wherein (C) is a mixture of an alkyl phosphite independently having from 1 to 20 carbon atoms in each alkyl group, and a triazole or derivative thereof.

18. A method of lubricating a refrigeration system comprising the steps of introducing into the refrigeration system a liquid composition prepared by a process of any one of claims 1 to 17; and operating the system.

## Patentansprüche (Patentansprüche für folgende(n) Vertragsstaat(en): AT, BE, CH, DE, DK, FR, GB, GR, IT, LI, LU, NL, SE, MC, PT, IE)

1. Eine flüssige Zusammensetzung, umfassend:
(A) mindestens einen Fluor-enthaltenden Kohlenwasserstoff mit 1 bis 3 Kohlenstoffatomen, und ein Schmiermittel, umfassend (B) ein Gemisch von
(1) mindestens einem Carbonsäureester von (I) einer Neohydroxyverbindung ausgewählt aus (i) einer Neohydroxyverbindung mit 1 bis 4 Hydroxylgruppen und (ii) einem Gemisch von Neohydroxyverbindungen mit durchschnittlich weniger als 3,5 Hydroxylgruppen pro Hydroxyverbindung und (II) einem Monocarbonsäureacylierungsmittel ausgewählt aus (i) einem Monocarbonsäureacylierungsmittel mit 4 oder 5 Kohlenstoffatomen und (ii) einer Kombination von (a) einem Monocarbonsäureacylierungsmittel mit 4 oder 5 Kohlenstoffatomen und (b) einem Monocarbonsäureacylierungsmittel mit 7 bis 15 Kohlenstoffatomen, mit der Maßgabe, daß mehr als 55% der Estergruppen von (1) abgeleitet sind von einem Monocubonsäureacylierungsmittel mit 4 oder 5 Kohlenstoffatomen, und
(2) mindestens einem Carbonsäureester von (III) einer Neohydroxyverbindung ausgewählt aus (iii) einer Neohydroxyverbindung mit mehr als 4 Hydroxylgruppen pro Hydroxyverbindung und (iv) einem Gemisch von Neohydroxyverbindungen mit durchschnittlich mehr als 4,5 Hydroxylgruppen pro Hydroxyverbindung und (IV) einer Kombination von (a) einem Monocarbonsäureacylierungsmittel mit 4 oder 5 Kohlenstoffatomen und (b) einem Monocarbonsäureacylierungsmittel mit 7 bis 15 Kohlenstoffatomen, mit der Maßgabe, daß mehr als 55% der Estergruppen von (2) abgeleitet sind von (IVb).

2. Flüssige Zusammensetzung nach Anspruch 1, wobei Fluor das einzige Halogen im Fluor-enthaltenden Kohlenwasserstoff (A) ist.

3. Flüssige Zusammensetzung nach einem der Ansprüche 1 oder 2, wobei der Fluorenthaltende Kohlenwasserstoff 1,1,1,2-Tetrafluorethan ist.

4. Flüssige Zusammensetzung nach einem der vorstehenden Ansprüche, wobei die Neohydroxyverbindung (I) ein Trimethylolalkan ist.

5. Flüssige Zusammensetzung nach einem der vorstehenden Ansprüche, wobei die Neohydroxyverbindung (I) Trimethylolpropan ist.

6. Flüssige Zusammensetzung nach einem der vorstehenden Ansprüche, wobei die Neohydroxyverbindung (III) durchschnittlich 5 oder mehr Hydroxylgruppen enthält.

7. Flüssige Zusammensetzung nach einem der vorstehenden Ansprüche, wobei die Neohydroxyverbindung (III) Dipentaerythrit oder Tripentaerythrit ist.

8. Flüssige Zusammensetzung nach einem der vorstehenden Ansprüche, wobei das Monocarbonsäureacylierungsmittel von (IIa) und (IVa) unabhängig Isobutan-, Pentan-, 2-Methylbutan- oder Neopentansäure oder das -säureanhydrid ist.

9. Flüssige Zusammensetzung nach einem der vorstehenden Ansprüche, wobei das Monocarbonsaureacylierungsmittel von (IIb) und (IVb) unabhängig ein verzweigtkettiges Monocarbonsäureacylierungsmittel mit 8 oder 9 Kohlenstoffatomen ist.

10. Flüssige Zusammensetzung nach einem der vorstehenden Ansprüche, des weiteren umfassend (C) mindestens ein Additiv ausgewählt aus einem Alkylphosphit, einem Alkylphosphonsäureester, einem Stickstoff-enthaltenden Heterocyclus und einem Gemisch davon.

11. Flüssige Zusammensetzung nach Anspruch 10, wobei (C) ein Alkylphosphit oder Alkylphosphonsäureester mit unabhängig 1 bis 20 Kohlenstoffatomen in jedem Alkylrest ist.

12. Flüssige Zusammensetzung nach Anspruch 10, wobei (C) ein Alkylphosphit oder Alkylphosphonsäureester mit unabhängig 3 bis 8 Kohlenstoffatomen in jedem Alkylrest ist.

13. Flüssige Zusammensetzung nach Anspruch 10, wobei (C) Dibutylphosphit ist.

14. Flüssige Zusammensetzung nach Anspruch 10, wobei (C) Dibutyl-butylphosphonat ist.

15. Flüssige Zusammensetzung nach Anspruch 10, wobei (C) ein Triazol oder ein Derivat davon ist.

16. Flüssige Zusammensetzung nach Anspruch 10, wobei (C) Tolyltriazol, Benzotriazol oder ein Reaktionsprodukt eines Benzotriazols und eines Amins und eines Aldehyds oder einer Aldehydvorstufe ist.

17. Flüssige Zusammensetzung nach Anspruch 10, wobei (C) ein Gemisch eines Alkylphosphits mit unabhängig 1 bis 20 Kohlenstoffatomen in jedem Alkylrest und einem Triazol oder Derivat davon ist.

18. Ein Verfahren zum Schmieren eines Kühlsystems, umfassend die Schritte des Einführens einer flüssigen Zusammensetzung nach einem der Ansprüche 1 bis 17 in das Kühlsystem und Betreiben des Systems.

## Patentansprüche (Patentansprüche für folgende(n) Vertragsstaat(en): ES)

1. Ein Verfahren zur Herstellung einer flüssigen Zusammensetzung, umfassend das Vermischen von:
(A) mindestens einem Fluor-enthaltenden Kohlenwasserstoff mit 1 bis 3 Kohlenstoffatomen, und einem Schmiermittel, umfassend (B) ein Gemisch von
(1) mindestens einem Carbonsäureester von (I) einer Neohydroxyverbindung ausgewählt aus (i) einer Neohydroxyverbindung mit 1 bis 4 Hydroxylgruppen und (ii) einem Gemisch von Neohydroxyverbindungen mit durchschnittlich weniger als 3,5 Hydroxylgruppen pro Hydroxyverbindung und (II) einem Monocarbonsäureacylierungsmittel ausgewählt aus (i) einem Monocarbonsäureacylierungsmittel mit 4 oder 5 Kohlenstoffatomen und (ii) einer Kombination von (a) einem Monocarbonsäureacylierungsmittel mit 4 oder 5 Kohlenstoffatomen und (b) einem Monocarbonsäureacylierungsmittel mit 7 bis 15 Kohlenstoffatomen, mit der Maßgabe, daß mehr als 55% der Estergruppen von (1) abgeleitet sind von einem Monocarbonsäureacylierungsmittel mit 4 oder 5 Kohlenstoffatomen, und
(2) mindestens einem Carbonsäureester von (III) einer Neohydroxyverbindung ausgewählt aus (iii) einer Neohydroxyverbindung mit mehr als 4 Hydroxylgruppen pro Hydroxyverbindung und (iv) einem Gemisch von Neohydroxyverbindungen mit durchschnittlich mehr als 4,5 Hydroxylgruppen pro Hydroxyverbindung und (IV) einer Kombination von (a) einem Monocarbonsäureacylierungsmittel mit 4 oder 5 Kohlenstoffatomen und (b) einem Monocarbonsäureacylierungsmittel mit 7 bis 15 Kohlenstoffatomen, mit der Maßgabe, daß mehr als 55% der Estergruppen von (2) abgeleitet sind von (IVb).

2. Verfahren nach Anspruch 1, wobei Fluor das einzige Halogen im Fluor-enthaltenden Kohlenwasserstoff (A) ist.

3. Verfahren nach einem der Ansprüche 1 oder 2, wobei der Fluor-enthaltende Kohlenwasserstoff 1,1,1,2-Tetrafluorethan ist.

4. Verfahren nach einem der vorstehenden Ansprüche, wobei die Neohydroxyverbindung (I) ein Trimethylolalkan ist.

5. Verfahren nach einem der vorstehenden Ansprüche, wobei die Neohydroxyverbindung (I) Trimethylolpropan ist.

6. Verfahren nach einem der vorstehenden Ansprüche, wobei die Neohydroxyverbindung (III) durchschnittlich 5 oder mehr Hydroxylgruppen enthält.

7. Verfahren nach einem der vorstehenden Ansprüche, wobei die Neohydroxyverbindung (III) Dipentaerythrit oder Tripentaerythrit ist.

8. Verfahren nach einem der vorstehenden Ansprüche, wobei das Monocarbonsäureacylierungsmittel von (IIa) und (IVa) unabhängig Isobutan-, Pentan-, 2-Methylbutan- oder Neopentansäure oder das -säureanhydrid ist.

9. Verfahren nach einem der vorstehenden Ansprüche, wobei das Monocarbonsäureacylierungsmittel von (IIb) und (IVb) unabhängig ein verzweigtkettiges Monocarbonsäureacylierungsmittel mit 8 oder 9 Kohlenstoffatomen ist.

10. Verfahren nach einem der vorstehenden Ansprüche, des weiteren umfassend (C) mindestens ein Additiv ausgewählt aus einem Alkylphosphit, einem Alkylphosphonsäureester, einem Stickstoff-enthaltenden Heterocyclus und einem Gemisch davon.

11. Verfahren nach Anspruch 10, wobei (C) ein Alkylphosphit oder Alkylphosphonsäureester mit unabhängig 1 bis 20 Kohlenstoffatomen in jedem Alkylrest ist.

12. Verfahren nach Anspruch 10, wobei (C) ein Alkylphosphit oder Alkylphosphonsäureester mit unabhängig 3 bis 8 Kohlenstoffatomen in jedem Alkylrest ist.

13. Verfahren nach Anspruch 10, wobei (C) Dibutylphosphit ist.

14. Verfahren nach Anspruch 10, wobei (C) Dibutyl-butylphosphonat ist.

15. Verfahren nach Anspruch 10, wobei (C) ein Triazol oder ein Derivat davon ist.

16. Verfahren nach Anspruch 10, wobei (C) Tolyltriazol, Benzotriazol, oder ein Reaktionsprodukt eines Benzotriazols und eines Amins und eines Aldehyds oder einer Aldehydvorstufe ist.

17. Verfahren nach Anspruch 10, wobei (C) ein Gemisch eines Alkylphosphits mit unabhängig 1 bis 20 Kohlenstoffatomen in jedem Alkylrest und eines Triazols oder Derivats davon ist.

18. Ein Verfahren zum Schmieren eines Kühlsystems, umfassend die Schritte des Einführens einer flüssigen Zusammensetzung, hergestellt nach einem Verfahren nach einem der Ansprüche 1 bis 17 in das Kühlsystem und Betreiben des Systems.

## Revendications (Revendications pour l'(les) Etat(s) contractant(s) suivant(s): AT, BE, CH, DE, DK, FR, GB, GR, IT, LI, LU, NL, SE, MC, PT, IE)

1. Une composition liquide comportant :
(A) au moins un hydrocarbure renfermant du fluor, qui contient de 1 à 3 atomes de carbone ; et un lubrifiant comportant (B) un mélange de
(1) au moins un ester carboxylique de (I) un composé néo-hydroxy, choisi parmi (i) un composé néo hydroxy ayant de un à quatre groupes hydroxyle, et (ii) un mélange de composés néo hydroxy ayant une moyenne d'au moins 3,5 groupes hydroxyle par composé hydroxy, et (II) un agent acylant monocarboxylique, choisi parmi (i) un agent acylant monocarboxylique ayant 4 ou 5 atomes de carbone, et (ii) une combinaison de (a) un agent acylant monocarboxylique, qui a 4 ou 5 atomes de carbone et, (b) un agent acylant monocarboxylique ayant de 7 à 15 atomes de carbone, sous la condition que plus de 55 % des groupes ester de (1) soient dérivés d'un agent acylant monocarboxylique, ayant 4 ou 5 atomes de carbone, et
(2) au moins un ester carboxylique de (III) un composé néo hydroxy choisi parmi (iii) un composé néo hydroxy ayant plus de quatre groupes hydroxyle par composé hydroxy, et (iv) un mélange de composés néo hydroxy, ayant une moyenne supérieure à 4,5 groupes hydroxyle par composé hydroxy, et (IV) une combinaison de (a) un agent acylant monocarboxylique ayant quatre ou cinq atomes de carbone et (b) un agent acylant monocarboxylique ayant de 7 à 15 atomes de carbone, sous la condition que plus de 55 % des groupes ester de (2) soient dérivés de (IVb).

2. Une composition liquide de la revendication 1, dans laquelle le fluor est le seul halogène dans l'hydrocarbure (A) renfermant du fluor.

3. Une composition liquide de l'une quelconque des revendications 1 et 2, dans laquelle l'hydrocarbure renfermant du fluor est le 1,1,1,2-tétrafluoroéthane.

4. Une composition liquide de l'une quelconque des revendications précédentes, dans laquelle le composé néo hydroxy (I) est un triméthylolalcane.

5. Une composition liquide de l'une quelconque des revendications précédentes, dans laquelle le composé néo hydroxy (I) est le triméthylolpropane.

6. Une composition liquide de l'une quelconque des revendications précédentes, dans laquelle le composé néo hydroxy (III) renferme une moyenne de 5 groupes hydroxyle ou plus.

7. Une composition liquide de l'une quelconque des revendications précédentes, dans laquelle le composé néo hydroxy (III) est le dipentaérythritol ou le tripentaérythritol.

8. Une composition liquide de l'une quelconque des revendications précédentes, dans laquelle l'agent acylant monocarboxylique de (IIa) et (IVa) est indépendamment un acide ou un anhydride isobutyrique valérique, 2-méthylbutyrique ou néopentanoïque.

9. Une composition liquide de l'une quelconque des revendications précédentes, dans laquelle l'agent acylant monocarboxylique de (IIb) et (IVb) est indépendamment un agent acylant monocarboxylique ramifié ayant 8 ou 9 atomes de carbone.

10. Une composition liquide de l'une quelconque des revendications précédentes, qui comporte en outre (C) au moins un additif choisi parmi un phosphite d'alkyle, un ester alkylique d'acide phosphonique, un hétérocycle renfermant de l'azote et un mélange de ceux-ci.

11. Une composition liquide de la revendication 10, dans laquelle (C) est un phosphite d'alkyle ou un ester alkylique d'acide phosphonique, qui possède indépendamment de 1 à 20 atomes de carbone dans chaque groupe alkyle.

12. Une composition liquide de la revendication 10, dans laquelle (C) est un phosphite d'alkyle ou un ester alkylique d'acide phosphonique, qui possède indépendamment de 3 à 8 atomes de carbone dans chaque groupe alkyle.

13. Une composition liquide de la revendication 10, dans laquelle (C) est du phosphite de dibutyle.

14. Une composition liquide de la revendication 10, dans laquelle (C) est un phosphonate de butyle ou de dibutyle.

15. Une composition liquide de la revendication 10, dans laquelle (C) est un triazole ou un dérivé de celui-ci.

16. Une composition liquide de la revendication 10, dans laquelle (C) est un tolyltriazole, un benzotriazole, ou un produit de la réaction d'un benzotriazole et d'une amine et d'une aldéhyde ou d'un précurseur d'aldéhyde.

17. Une composition liquide de la revendication 10, dans laquelle (C) est un mélange d'un phosphite d'alkyle, qui possède indépendamment de 1 à 20 atomes de carbone dans chaque groupe alkyle, et d'un triazole ou d'un dérivé de celui-ci.

18. Un procédé de lubrification d'un système de réfrigération, qui comporte les étapes d'introduire dans le système de réfrigération une composition liquide de l'une quelconque des revendications 1 à 17 ; et de faire fonctionner le système.

## Revendications (Revendications pour l'(les) Etat(s) contractant(s) suivant(s): ES)

1. Un procédé pour la préparation d'une composition liquide consistant à mélanger :
(A) au moins un hydrocarbure renfermant du fluor, qui contient de 1 à 3 atomes de carbone ; et un lubrifiant comportant (B) un mélange de
(1) au moins un ester carboxylique de (I) un composé néo--hydroxy, choisi parmi (i) un composé néo hydroxy ayant de un à quatre groupes hydroxyle, et (ii) un mélange de composés néo hydroxy ayant une moyenne d'au moins 3,5 groupes hydroxyle par composé hydroxy, et (II) un agent acylant monocarboxylique, choisi parmi (i) un agent acylant monocarboxylique ayant 4 ou 5 atomes de carbone, et (ii) une combinaison de (a) un agent acylant monocarboxylique, qui a 4 ou 5 atomes de carbone et, (b) un agent acylant monocarboxylique ayant de 7 à 15 atomes de carbone, sous la condition que plus de 55 % des groupes ester de (1) soient dérivés d'un agent acylant monocarboxylique, ayant 4 ou 5 atomes de carbone, et
(2) au moins un ester carboxylique de (III) un composé néo hydroxy choisi parmi (iii) un composé néo hydroxy ayant plus de quatre groupes hydroxyle par composé hydroxy, et (iv) un mélange de composés néo hydroxy, ayant une moyenne supérieure à 4,5 groupes hydroxyle par composé hydroxy, et (IV) une combinaison de (a) un agent acylant monocarboxylique ayant quatre ou cinq atomes de carbone et (b) un agent acylant monocarboxylique ayant de 7 à 15 atomes de carbone, sous la condition que plus de 55 % des groupes ester de (2) soient dérivés de (IVb).

2. Un procédé de la revendication 1, dans lequel le fluor est le seul halogène dans l'hydrocarbure (A) renfermant du fluor.

3. Un procédé de l'une quelconque des revendications 1 et 2, dans lequel l'hydrocarbure renfermant du fluor est le 1,1,1,2-tétrafluoroéthane.

4. Un procédé de l'une quelconque des revendications précédentes, dans lequel le composé néo hydroxy (I) est un triméthylolalcane.

5. Un procédé de l'une quelconque des revendications précédentes, dans lequel le composé néo hydroxy (I) est le triméthylolpropane.

6. Un procédé de l'une quelconque des revendications précédentes, dans lequel le composé néo hydroxy (III) renferme une moyenne de 5 groupes hydroxyle ou plus.

7. Un procédé de l'une quelconque des revendications précédentes, dans lequel le composé néo hydroxy (III) est le dipentaérythritol ou le tripentaérythritol.

8. Un procédé de l'une quelconque des revendications précédentes, dans lequel l'agent acylant monocarboxylique de (IIa) et (IVa) est indépendamment un acide ou un anhydride isobutyrique, valérique, 2-méthylbutyrique ou néopentanoïque.

9. Un procédé de l'une quelconque des revendications précédentes, dans lequel l'agent acylant monocarboxylique de (IIb) et (IVb) est indépendamment un agent acylant monocarboxylique ramifié ayant 8 ou 9 atomes de carbone.

10. Un procédé de l'une quelconque des revendications précédentes, qui comporte en outre (C) au moins un additif choisi parmi un phosphite d'alkyle, un ester alkylique d'acide phosphonique, un hétérocycle renfermant de l'azote et un mélange de ceux-ci.

11. Un procédé de la revendication 10, dans lequel (C) est un phosphite d'alkyle ou un ester alkylique d'acide phosphonique, qui possède indépendamment de 1 à 20 atomes de carbone dans chaque groupe alkyle.

12. Un procédé de la revendication 10, dans lequel (C) est un phosphite d'alkyle ou un ester alkylique d'acide phosphonique, qui possède indépendamment de 3 à 8 atomes de carbone dans chaque groupe alkyle.

13. Un procédé de la revendication 10, dans lequel (C) est du phosphite de dibutyle.

14. Un procédé de la revendication 10, dans lequel (C) est un phosphonate de butyle ou de dibutyle.

15. Un procédé de la revendication 10, dans lequel (C) est un triazole ou un dérivé de celui-ci.

16. Un procédé de la revendication 10, dans lequel (C) est un tolyltriazole, un benzotriazole, ou un produit de la réaction d'un benzotriazole et d'une amine et d'une aldéhyde ou d'un précurseur d'aldéhyde.

17. Un procédé de la revendication 10, dans lequel (C) est un mélange d'un phosphite d'alkyle, qui possède indépendamment de 1 à 20 atomes de carbone dans chaque groupe alkyle, et d'un triazole ou d'un dérivé de celui-ci.

18. Une méthode de lubrification d'un système de réfrigération, comportant les étapes d'introduire dans le système de réfrigération une composition liquide préparée par un procédé selon l'une quelconque des revendications 1 à 17, et de faire fonctionner le système.
